# EUROPÄISCHE PATENTSCHRIFT

(11) **EP 0 683 157 B1**
(45) Veröffentlichungstag und Bekanntmachung des Hinweises auf die Patenterteilung: **26.05.2004**
(21) Anmeldenummer: 95111736.5
(22) Anmeldetag: 29.09.1990
(51) Int. Cl.: C07D 249/12

(54) **Sulfonylaminocarbonyltriazolinone als Herbizide**
Sulfonylaminocarbonyltriazolinones as herbicides
Sulfonylaminocarbonyltriazolinones comme herbicides

(30) Priorität: 12.10.1989 DE 3934081
(43) Veröffentlichungstag der Anmeldung: 22.11.1995
(62) Teilanmeldung aus: 90118750.0
(73) Patentinhaber: Bayer CropScience AG, 40789 Monheim (DE)
(72) Erfinder: Müller, Klaus-Helmut, Dr., D-40593 Düsseldorf (DE); Babczinski, Peter, Dr., D-42115 Wuppertal (DE); Santel, Hans-Joachim, Dr., D-51371 Leverkusen (DE); Schmidt, Robert R., Dr., D-51467 Bergisch Gladbach (DE)

(56) Entgegenhaltungen:
- Keine einschlägigen Dokumente gefunden

## Beschreibung

Die Erfindung betrifft Zwischenprodukte für die Herstellung neuer Sulfonylaminocarbonyltriazolinone.

Es ist bekannt, dass bestimmte substituierte Aminocarbonylimidazolidinone, wie z.B. 1-Isobutylaminocarbonyl-2-imidazolidinon (Isocarbamid), herbizide Eigenschaften aufweisen (vgl. R. Wegler, Chemie der Pflanzenschutz- und Schädlingsbekämpfungsmittel, Band 5, S. 219, Springer-Verlag, Berlin-Heidelberg-New York, 1977). Die Wirkung dieser Verbindung ist jedoch nicht in allen Belangen zufriedenstellend.

Es wurden nun die neuen Sulfonylaminocarbonyl-triazolinone der allgemeinen Formel (I), 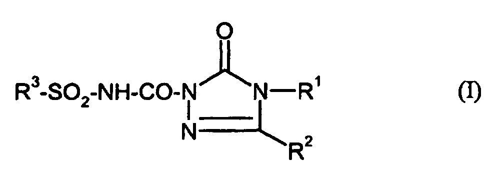 in welcher
- R¹: für Allyl, für C₃-C₆-Cycloalkyl, für Phenyl, für Benzyl, für C₁-C₃-Alkoxy, für C₁-C₃ -Alkylamino oder für Di-(C₁-C₂-alkyl)-amino steht,
- R²: für gegebenenfalls durch Fluor und/oder Chlor, Methoxy oder Ethoxy substituiertes C₁-C₄-Alkyl, für C₃-C₆-Cycloalkyl, für Phenyl, für C₁-C₃-Alkoxy, für C₁-C₃-Alkylamino oder für Di-(C₁-C₂-alkyl)-amino steht, und
- R3: für die Gruppierung 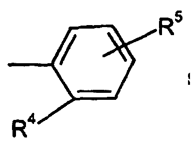 steht, worin
R⁴ für Fluor, Chlor, Brom, Methyl, Trifluormethyl, Methoxy, Difluormethoxy, Trifluormethoxy, 2-Chlor-ethoxy, 2-Methoxy-ethoxy, C₁-C₃-Alkylthio, C₁-C₃-Alkylsulfinyl, C₁-C₃-Alkylsulfonyl, Dimethylaminosulfonyl, Diethylaminosulfonyl, N-Methoxy-N-methylaminosulfonyl, Phenyl, Phenoxy oder C₁-C₃-Alkoxy-carbonyl steht und
R⁵ für Wasserstoff, Fluor, Chlor oder Brom steht;
weiterhin
- R³: für den Rest 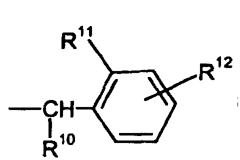 steht, worin
R¹⁰ für Wasserstoff steht,
R¹¹ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
R¹² für Wasserstoff steht;
weiterhin
- R3: für den Rest 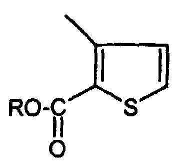 steht,
worin R für C₁-C₄-Alkyl steht, oder
für den Rest 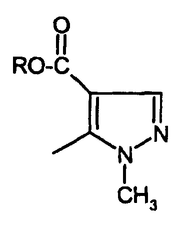 steht,
worin R für C₁-C₄-Alkyl steht,
sowie Salze von Verbindungen der Formel (I) gefunden,
ausgenommen jedoch - für die Vertragsstaaten BE, CH, DE, FR, GB, IT, LI und NL - die Verbindungen:
4-Cyclopropyl-5-methyl-2-(2-methoxycarbonyl-phenylsulfonyl-aminocarbonyl)-2,4-dihydro-3H-1,2,4-triazol-3-on
   und
4-Dimethylamino-5-methyl-2-(2-methoxycarbonyl-phenylsulfonyl-aminocarbonyl)-2,4-dihydro-3H-1,2,4-triazol-3-on.

Man erhält einen Teil der neuen Sulfonylaminocarbonyltriazolinone der allgemeinen Formel (II) wenn man
a) Triazolinone der allgemeinen Formel (II) 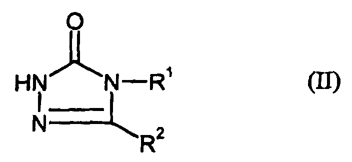 in welcher
   - R¹: für C₁-C₃-Alkoxy oder Cyclopropyl steht und

   (i) wenn R¹ C₁-C₃-Alkoxy bedeutet,
      - R²: für gegebenenfalls durch Fluor und/oder Chlor, Methoxy oder Ethoxy substituiertes C₁-C₄-Alkyl oder für Cyclopropyl steht,
      oder
   (ii) wenn R¹ Cyclopropyl bedeutet,
      - R²: für Propyl, Isopropyl, Butyl, Cyclopropyl oder Methoxymethyl steht,
      mit Sulfonylisocyanaten der allgemeinen Formel (III)

      R³-SO₂-N=C=O (III)

      in welcher
      - R³: die oben angegebene Bedeutung hat,
      gegebenenfalls in Gegenwart eines Verdünnungsmittel umsetzt, oder wenn man
   b) Triazolinone der allgemeinen Formel (II) 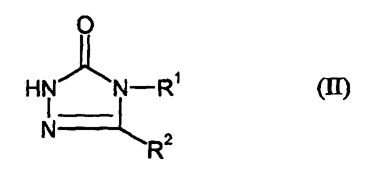 in welcher
      R¹ und R² die unter a) angegebenen Bedeutungen haben,
      mit Sulfonsäureamid-Derivaten der allgemeinen Formel (VI)

      R³-SO₂-NH-CO-Z (VI)

      in welcher
      - R³: die oben angegebene Bedeutung hat und
      - Z: für Chlor, C₁-C₄-Alkoxy, Benzyloxy oder Phenoxy steht,
      gegebenenfalls in Gegenwart eines Säureakzeptors und gegebenenfalls in Gegenwart eines Verdünnungsmittels umsetzt und gegebenenfalls aus den nach Verfahren (a), (b) oder (c) hergestellten Verbindungen der Formel (I) nach üblichen Methoden Salze erzeugt.

Die neuen Sulfonylaminocarbonyltriazolinone der allgemeinen Formel (I) und ihre Salze zeichnen sich durch starke herbizide Wirksamkeit aus.

Überraschenderweise zeigen die neuen Verbindungen der Formel (I) erheblich bessere herbizide Wirkung als das strukturell ähnliche bekannte Herbizid 1-Isobutylamino-carbonyl-2-imidazolidinon (Isocarbamid).

Beispiele für die Verbindungen der Formel (I) sind in der nachstehenden Tabelle 1 aufgeführt - vgl. auch die Herstellungsbeispiele.

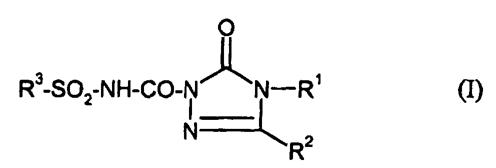

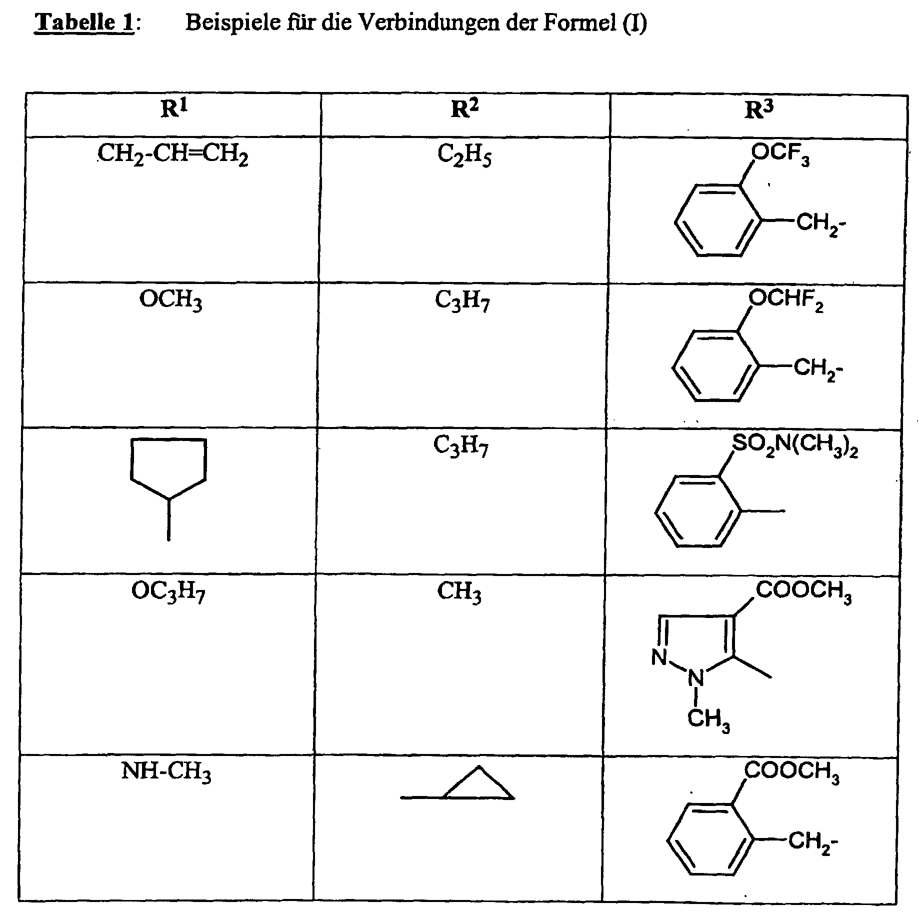

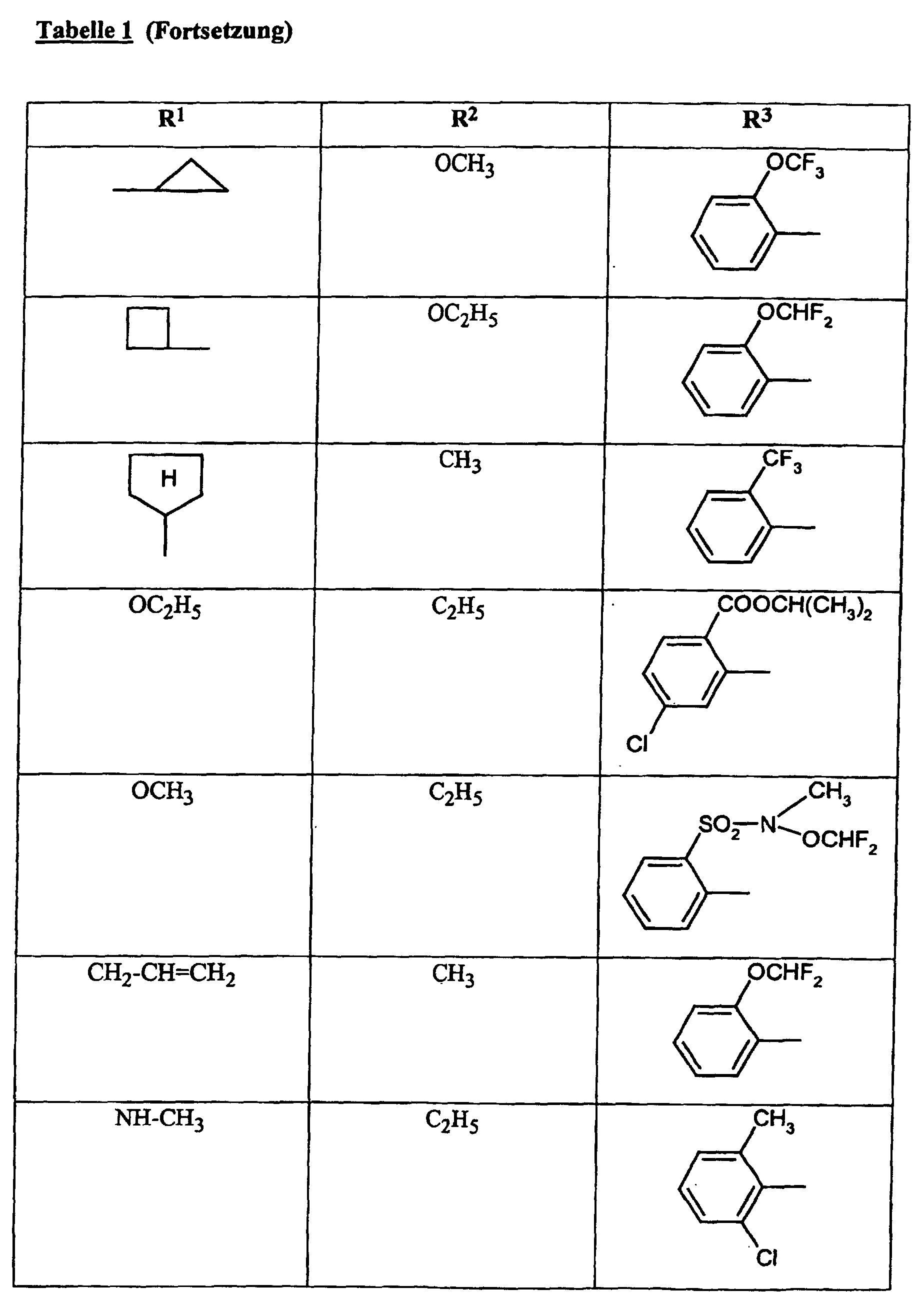

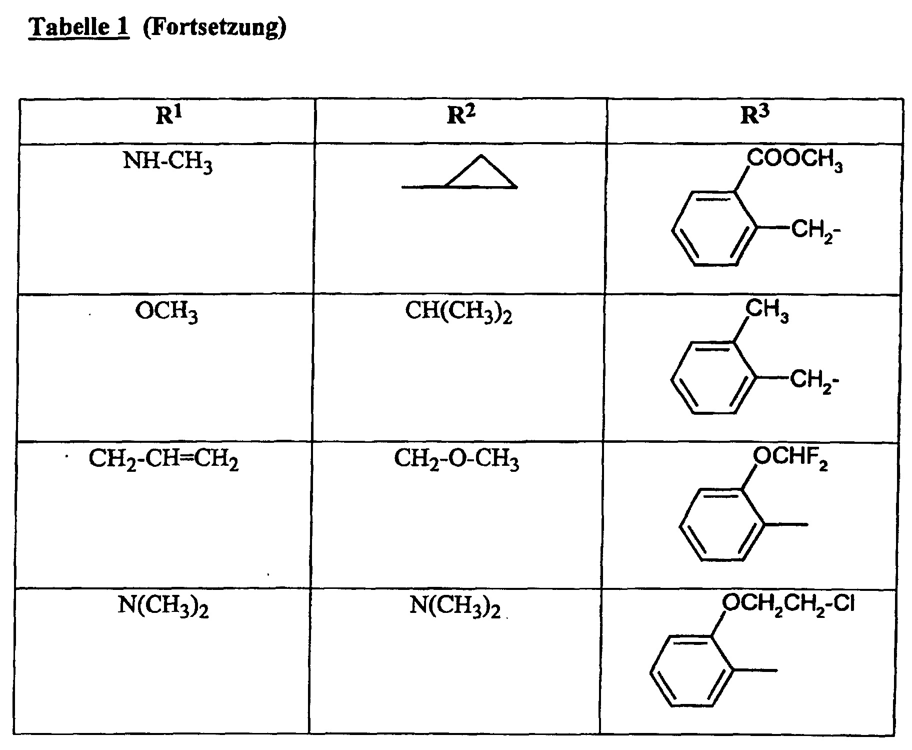

Verwendet man beispielsweise 2,6-Difluor-phenylisocyanat und 5-Ethyl-4-methoxy-2,4-dihydro-3H-1,2,4-triazol-3-on als Ausgangsstoffe, so kann der Reaktionsablauf beim erfindungsgemäßen Verfahren a) durch folgendes Formelschema skizziert werden:

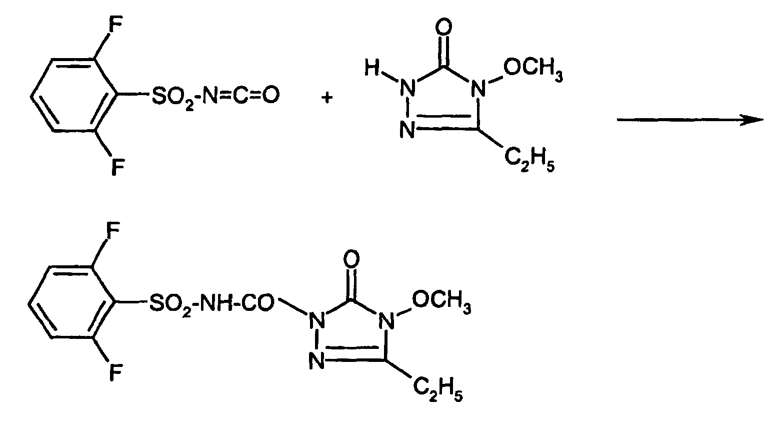

Verwendet man beispielsweise N-Methoxycarbonyl-2,6-difluor-benzosulfon-säureamid und 5-Ethyl-4-methoxy-2,4-dihydro-3H-1,2,4-triazol-3-on als Ausgangsstoffe, so kann der Reaktionsablauf beim erfindungsgemäßen Verfahren b) durch folgendes Formelschema skizziert werden:

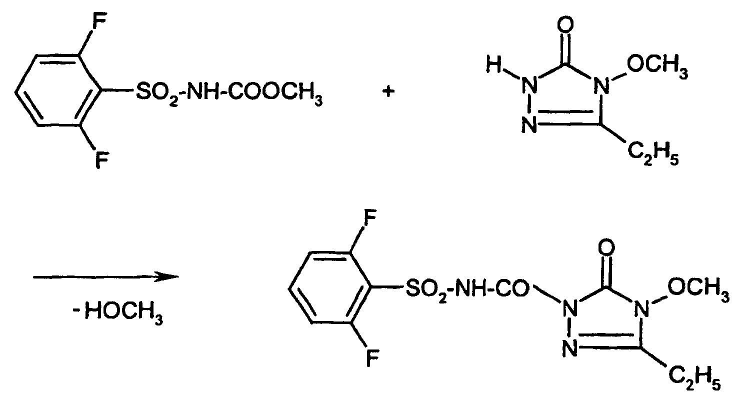

Die bei den Verfahren (a) und (b) zur Herstellung von Verbindungen der Formel (I) als Ausgangsstoffe zu verwendenden Triazolinone sind durch die Formel (II) allgemein definiert.

Beispiele für die Ausgangsstoffe der Formel (II) sind in der nachstehenden Tabelle 2 aufgeführt.

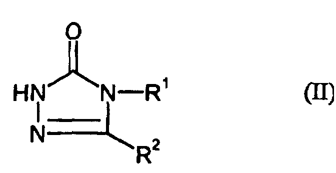

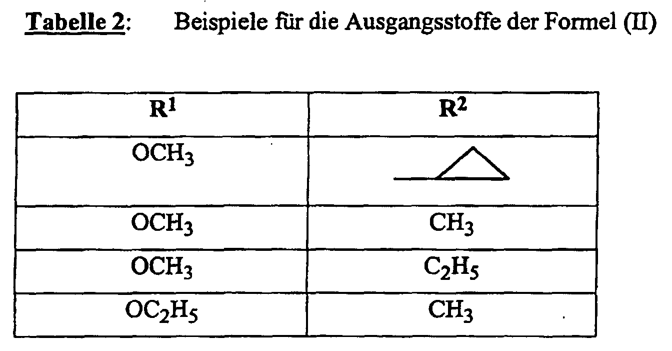

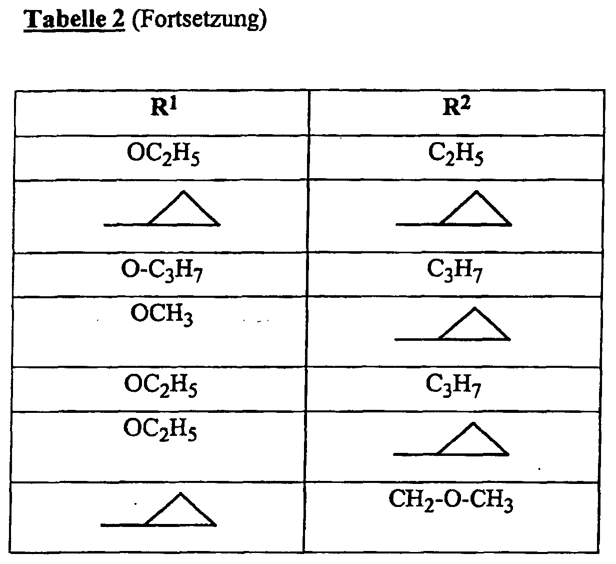

Die Ausgangsstoffe der Formel (II) sind neu und Gegenstand der Erfindung. Sie können nach an sich bekannten Verfahren hergestellt werden (vgl. Chem. Ber. 90 (1957), 909-921; ibid. 98 (1965), 3025-3099; J. Heterocycl. Chem. 15 (1978), 237-240; Tetrahedron 32 (1976), 2347-2352; Helv. Chim. Acta 63 (1980), 841-859; J. Chem. Soc. C 1967, 746-751; EP-A 28 38 76; EP-A 29 46 66, EP-A 30 19 46; EP-A 29 83 71; DE-P 3 839 206 vom 19.11.1988; DE-P 3 916 207 vom 18.05.1989; DE-P 3 916 208 vom 18.05.1989; J. Chem. Soc. C 1970, 26-34; DE-P 3 916 930 vom 24.05.1989).

Man erhält die Triazolinone der Formel (II) beispielsweise, wenn man
α) Oxadiazolinone der allgemeinen Formel (VII) 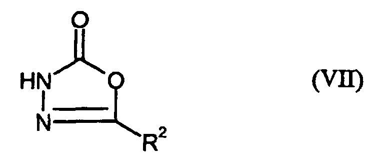 in welcher
   - R²: die in Formel (II) angegebene Bedeutung hat,
   mit Aminoverbindungen der allgemeinen Formel (VIII)

   H₂N-R¹ (VIII)

   in welcher
   - R¹: die in Formel (II) angegebene Bedeutung hat,
   gegebenenfalls in Gegenwart eines Verdünnungsmittels, wie z.B. Wasser, bei Temperaturen zwischen 20°C und 120°C umsetzt und die hierbei gebildeten Hydrazinderivate der allgemeinen Formel (IX) 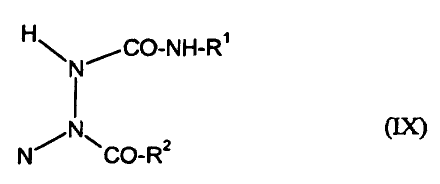 in welcher
   - R¹ und R²: die in Formel (II) angegebenen Bedeutungen haben,
   nach üblichen Methoden isoliert (vgl. die Herstellungsbeispiele) und - oder gegebenenfalls auch ohne Zwischenisolierung - die Verbindungen der Formel (IX), gegebenenfalls in Gegenwart eines basischen Kondensationshilfsmittels, wie z.B. Natriumhydroxid, und gegebenenfalls in Gegenwart eines Verdünnungsmittels, wie z.B. Wasser, bei Temperaturen zwischen 20°C und 120°C zu den Verbindungen der Formel (II) kondensiert (vgl. EP-A 301 936, DE-OS 3 743 493 und die Herstellungsbeispiele, oder wenn man
β) Aminoverbindungen der allgemeinen Formel (VIII)

   H₂N-R¹ (VIII)

   in welcher
   - R¹: die in Formel (II) angegebene Bedeutung hat,
   mit Kohlensäurederivaten, wie z.B. Diphenylcarbonat, anschließend mit Hydrazin oder Hydrazinhydrat und schließlich mit einem Carbonsäure- bzw. Kohlensäure-Derivat der allgemeinen Formel (X)

   (RO)₃C-R² (X)

   in welcher
   - R²: die in Formel (II) angegebene Bedeutung hat,
   - R: für Niederalkyl steht,
   gegebenenfalls in Gegenwart eines Verdünnungsmittels, wie z.B. Ethylenchlorid, bei Temperaturen zwischen 0°C und 150°C umsetzt (vgl. DE-P 3 920 270 vom 21.06.1989, DE-P 3 928 662 vom 30.08.1989 und die Herstellungsbeispiele.

Die zur Herstellung der Triazolinone der Formeln (II) als Ausgangsstoffe zu verwendenden Verbindungen der Formeln (VII), (VIII) und (X) sind bekannt (vgl. Helv. Chim. Acta 55 (1972), 1174; EP-A 301 946; DE-OS 3 743 493).

Die beim erfindungsgemäßen Verfahren (a) zur Herstellung von Verbindungen der Formel (I) weiter als Ausgangsstoffe zu verwendenden Sulfonylisocyanate sind durch die Formel (III) allgemein definiert.

Als Beispiele für die Ausgangsstoffe der Formel (III) seien genannt:
2-Fluor-, 2-Chlor-, 2-Brom-, 2-Methyl-, 2-Methoxy-, 2-Trifluormethyl-, 2-Difluormethoxy-, 2-Trifluormethoxy-, 2-Metylthio-, 2-Ethylthio-, 2-Propylthio-, 2-Methylsulfinyl-, 2-Methylsulfonyl-, 2-Dimethylaminosulfonyl-, 2-Diethylaminosulfonyl-, 2-(N-Methoxy-N-methyl)-aminosulfonyl-, 2-Phenyl-, 2-Phenoxy-, 2-Methoxycarbonyl-, 2-Ethoxycarbonyl-, 2-Propoxycarbonyl- und 2-Isopropoxycarbonylphenylsulfonylisocyanat, 2-Fluor-, 2-Chlor-, 2-Difluormethoxy-, 2-Trifluormethoxy-, 2-Methoxycarbonyl- und 2-Ethoxycarbonyl-benzylsulfonylisocyanat, 2-Methoxycarbonyl-3-thienyl-sulfonylisocyanat, 4-Methoxycarbonyl- und 4-Ethoxycarbonyl-1-methyl-pyrazol-5-yl-sulfonylisocyanat.

Die Sulfonylisocyanate der Formel (III) sind bekannt und/oder können nach an sich bekannten Verfahren hergestellt werden (vgl. US-P 4 127 405, 4 169 719, 4 371 391; EP-A 7 687, 13 480, 21 641, 23 141, 23 422, 30 139, 35 893, 44 808,44 809, 48 143, 51 466,64 322,70 041, 173 312).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Keton wie Aceton, Methyl-ethyl-, Methyl-isopropyl- und Methyl-isobutyl-keton, Ester wie Essigsäuremethylester und - ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren (a) in einem größeren Bereich variiert werden. Im Allgemeinen arbeitet man bei Temperaturen zwischen 0°C und 150°C, vorzugsweise bei Temperaturen zwischen 10°C und 80°C.

Das erfindungsgemäße Verfahren (a) wird im Allgemeinen bei Normaldruck durchgeführt.

Zur Durchführung des erfindungsgemäßen Verfahrens (a) setzt man je Mol Triazolinon der Formel (II) im Allgemeinen zwischen 1 und 3 mol, vorzugsweise zwischen 1 und 2 mol, Sulfonylisocyanat der Formel (III) ein.

Die Reaktionskomponenten können in beliebiger Reihenfolge zusammengegeben werden. Das Reaktionsgemisch wird bis zum Ende der Umsetzung gerührt, eingeengt und das im Rückstand verbleibende Rohprodukt mit einem geeigneten Lösungsmittel, wie z.B. Diethylether, zur Kristallisation gebracht. Das kristallin angefallene Produkt der Formel (I) wird durch Absaugen isoliert.

Die beim erfindungsgemäßen Verfahren (b) zur Herstellung von Verbindungen der Formel (I) als Ausgangsstoffe zu verwendenden Triazolinone der Formel (II) sind bereits als Ausgangsstoffe für das erfindungsgemäße Verfahren (a) beschrieben worden.

Die beim erfindungsgemäßen Verfahren (b) zur Herstellung von Verbindungen der Formel (I) weiter als Ausgangsstoffe zu verwendenden Sulfonsäureamid-Derivate sind durch die Formel (VI) allgemein definiert.

Das erfindungsgemäße Verfahren (b) wird vorzugsweise unter Verwendung von Verdünnungsmitteln durchgeführt. Es kommen hierbei die gleichen organischen Lösungsmittel in Betracht, die oben im Zusammenhang mit der Beschreibung des erfindungsgemäßen Verfahrens (a) genannt wurden.

Verfahren (b) wird gegebenenfalls in Gegenwart eines Säureakzeptors durchgeführt.

Die Reaktionstemperaturen können bei dem erfindungsgemäßen Verfahren (b) in einem größeren Beeich variiert werden. Im Allgemeinen arbeitet man bei Temperaturen zwischen 0°C und 100°C, vorzugsweise bei Temperaturen zwischen 10°C und 60°C.

Das erfindungsgemäße Verfahren (b) wird im Allgemeinen unter Normaldruck durchgeführt. Es ist jedoch auch möglich, unter erhöhten oder vermindertem Druck zu arbeiten.

Die Wirkstoffe der Formel (I) können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaeum, Sphenoclea, Dactyloctenium, Agrostis, Aleopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der Wirkstoffe ist jedoch keineswegs auf diese Gattungen beschränkt, sondern erstreckt sich in gleicher Weise auch auf andere Pflanzen.

Die Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die Verbindungen der Formel (I) eignen sich zur Bekämpfung von monokotylen und dikotylen Unkräutern sowohl im Vorauflauf - als auch im Nachauflauf-Verfahren. Sie sind deutlich wirksamer als z.B. Isocarbamid.

In gewissem Umfang zeigen die Verbindungen auch fungizide Wirkung, z.B. gegen echte Mehltaupilze und gegen Apfelschorf sowie gegen Pyricularia oryzae an Reis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im Wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5,-triazin-2,4-(1H, 3H)-dion (AMETHYDIONE) oder N-(2-Benzthiazolyl)-N,N'-dimethyl-hamstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfing in Zuckerrüben und 4-Amino-6-(1,1-dimethylethyl)-3-methyl-thio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitro-benzoesäure (ACIFLUORFEN); Chloressigsäure-N-(methoxy-methyl)-2,6-diethylanilid (ALACHLOR); 2-Chlor-4-ethylamino-6-ispropylamino-1,3,5-triazin (ATRAZIN); 2-[[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorpehnoxy)-2-nitro-benzoat (BIFENOX); 3,5-Dibrom-4-hydroxy-benzonitril (BROMOXYNIL); N-(Butoxymethyl)-2-chlor-N-(2,6-diethyl-phenyl)-acetamid (BUTACHLOR); Ethyl-2-{[(4-chlor-6-methoxy-2-pyrimidinyl)-aminocarbonyl]-aminosulfonyl}-benzoat (CHLOR-IMURON); 2-Chlor-N-{[(4-methoxy-6-methyl-1,3,5-triazin-2-yl)-amino]-carbonyl}benzolsulfonamid (CHLORSULFURON); N,N-Dimethyl-N'-(3-chlor-4-methylphenyl)-hamstoff (CHLORTOLURON); 2-Chlor-4-ethylamino-6-(3-cyanopropylamino)-1,3,5-triazin (CYANAZIN); 2,6-Dichlorbenzonitril (DICHLOBENIL); 2-[4-(2,4-Dichlorphenoxy)-phenoxy]-propionsäure, deren Methyl- oder deren Ethylester (DICLOFOP); 2-[(2-Chlorphenyl)-methyl]-4,4-dimethylisooxolidin-3-on (DIMETHAZONE); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Ethylester (FENOXAPROP); 2-[-4-(5-Trifluormethyl-2-pyridyloxy)-phenoxy]-propansäure oder deren Butylester (FLUAZIFOP); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); 5-(2-Chlor-4-trifluormethyl-phenoxy)-N-methylsulfonyl-2-nitrobenzamid (FOMESAFEN); N-Phosphonomethyl-glycin (GLYPHOSATE); Methyl-2-[4,5-dihydro-4-methyl-4-(1-methylethyl)-5-oxo-1Himidazol-2-yl]-4(5)-methylbenzoat (IMAZAMETHABENZ); 2-(4,5-Dihydro-4-methyl-4-isopropyl-5-oxo-1H-imidazol-2-yl)-pyridin-3-carbonsäure (IMAZAPYR); 2-[5-Methyl-5-( 1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZA-CHLOR); 2-Ethyl-6-methyl-N-(1-methyl-2-methoxyethyl)-chloracetanilid (METOLACHLOR); 2- {[[((4-Methoxy-6-methyl-1,3,5-triazin-2-yl)-amino)-carbonyl]-amino]-sulfonyl}-benzoesäure oder deren Methylester (MET-SULFURON); 1-(3-Trifluormethyl-phenyl)-4-methylamino-5-chlor-6-pyridazon (NORFLURAZON); N-(1-Ethylpropyl)-3,4-dimethyl-2,6-dinitroanilin (PENDI-METHALIN); O-(6-Chlor-3-phenyl-pyridazin-4-yl)-S-octyl-thiocarbamat (PYRI-DATE); 2-[4-(6-Chlor-chinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); Methyl-2-{[(4,6-dimethyl-2-pyrimidinyl)-aminocarbonyl]-aminosulfonyl}-benzoat (SULFOMETURON); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester THIAMETURON); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im Wesentlichen von der Art des gewünschten Effektes ab. Im Allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

### Herstellungsbeispiele

### Beispiel 1

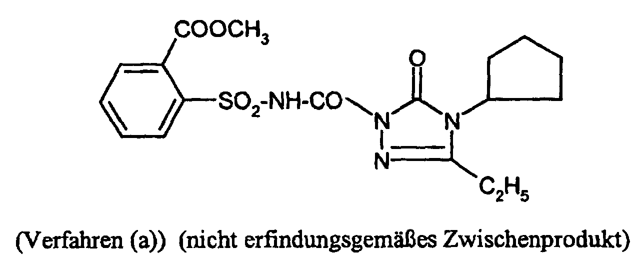

3,0 g (17,95 mmol) 4-Cyclopentyl-5-methyl-2,4-dihydro-3H-1,2,4-triazol-3-on werden in 60 ml Acetonitril gelöst und unter Rühren werden 6,9 g (28,6 mmol) 2-Methoxycarbonyl-phenylsulfonylisocyanat, gelöst in 20 ml Acetonitril zu dieser Lösung gegeben. Das Reaktionsgemisch wird 6 Stunden bei 20°C gerührt und dann eingeengt. Der verbleibende Rückstand wird mit Diethylether verrührt und das kristallin angefallende Produkt durch Absaugen isoliert.

Man erhält 6,6 g (90 % der Theorie) 4-Cyclopentyl-5-methyl-2-(2-methoxycarbonylphenylsulfonyl-aminocarbonyl)-2,4-dihydro-3H-1,2,4-triazol-3-on vom Schmelzpunkt 146°C.

Analog zu Beispiel 1 sowie entsprechend der allgemeinen Beschreibung der erfindungsgemäßen Herstellungsverfahren können beispielsweise auch die in der nachstehenden Tabelle 3 aufgeführten Verbindungen der Formel (I) hergestellt werden.

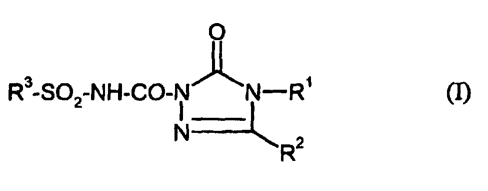

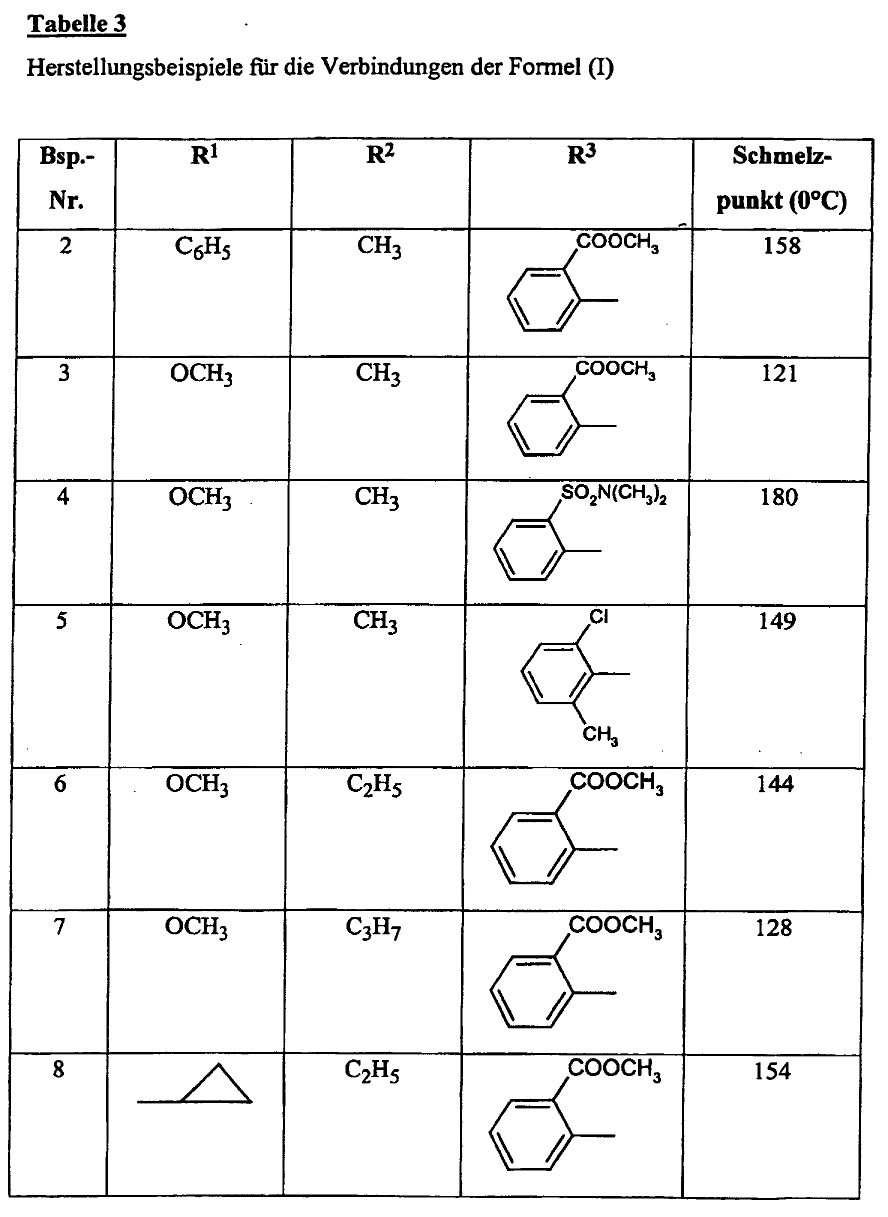

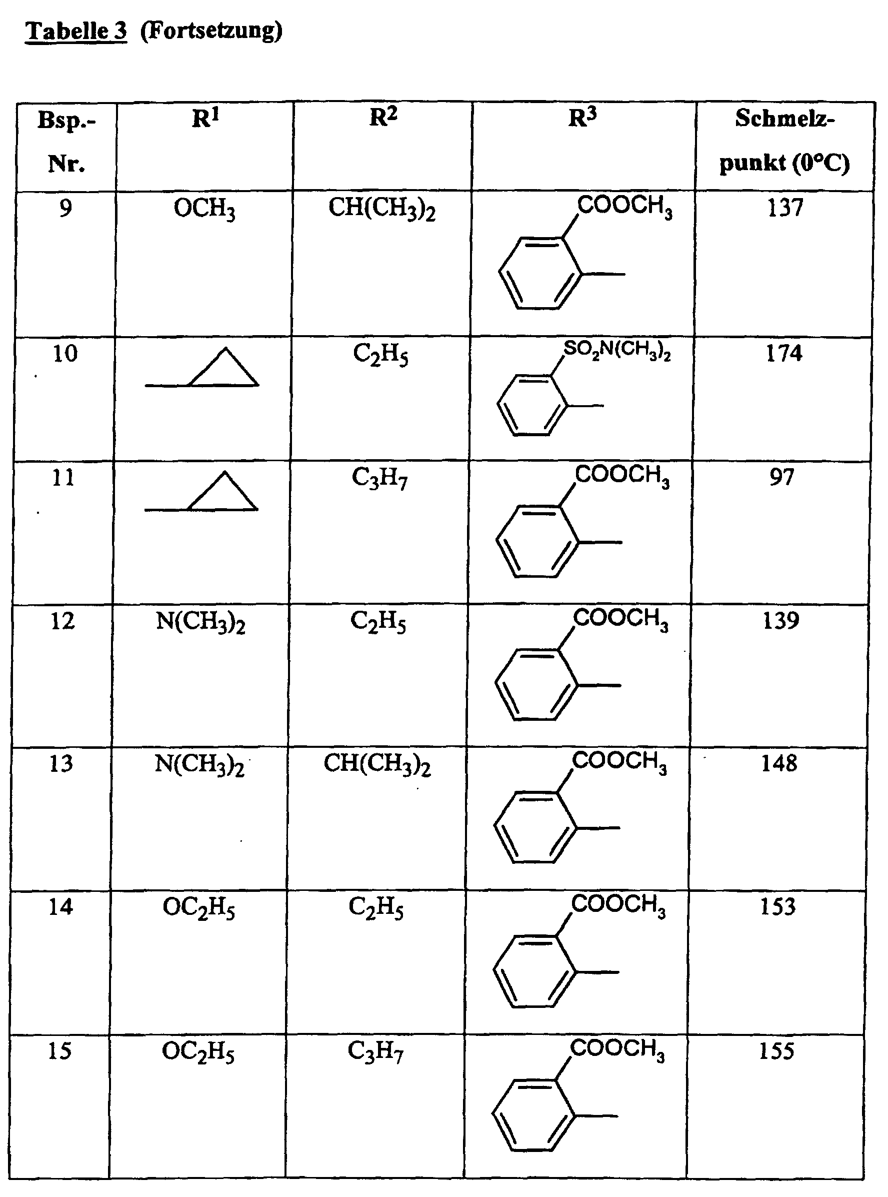

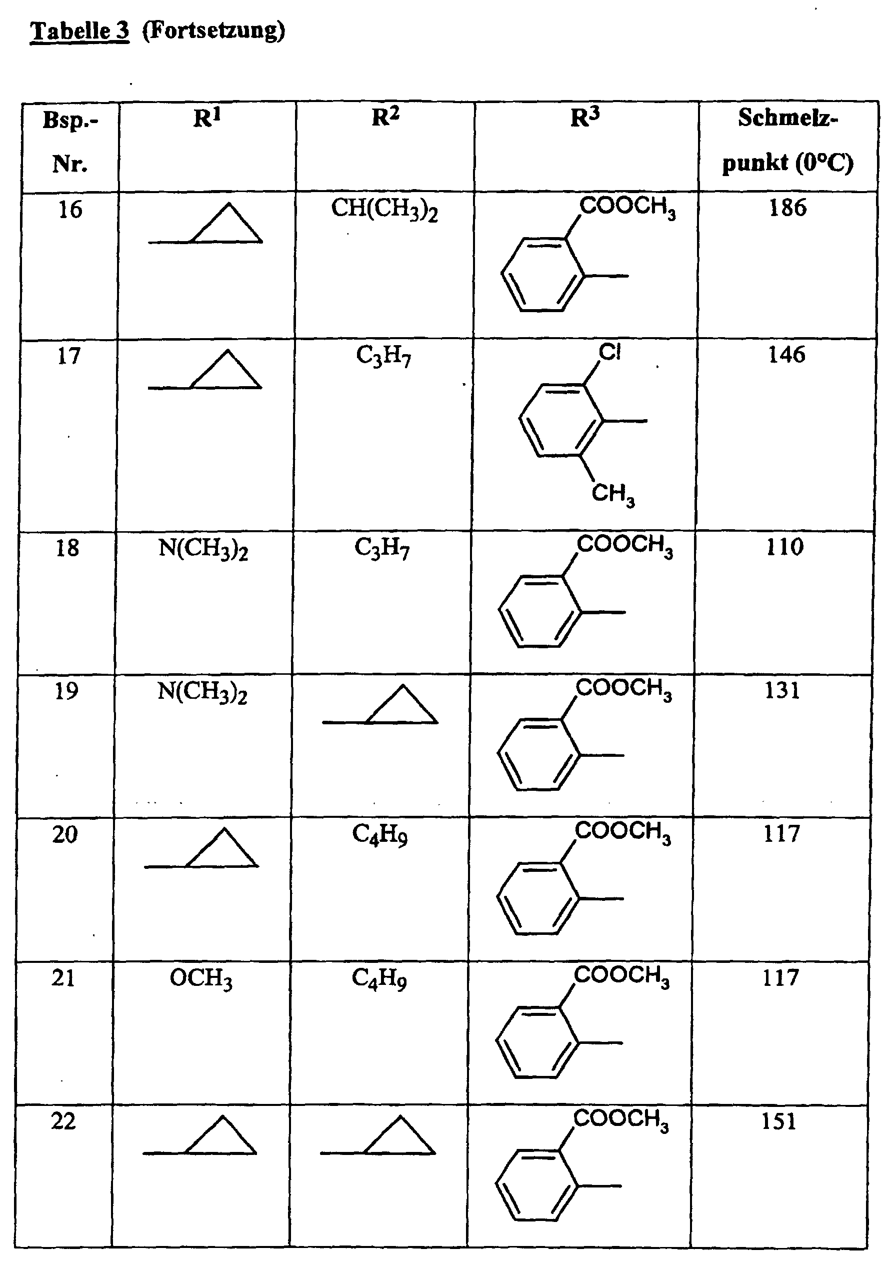

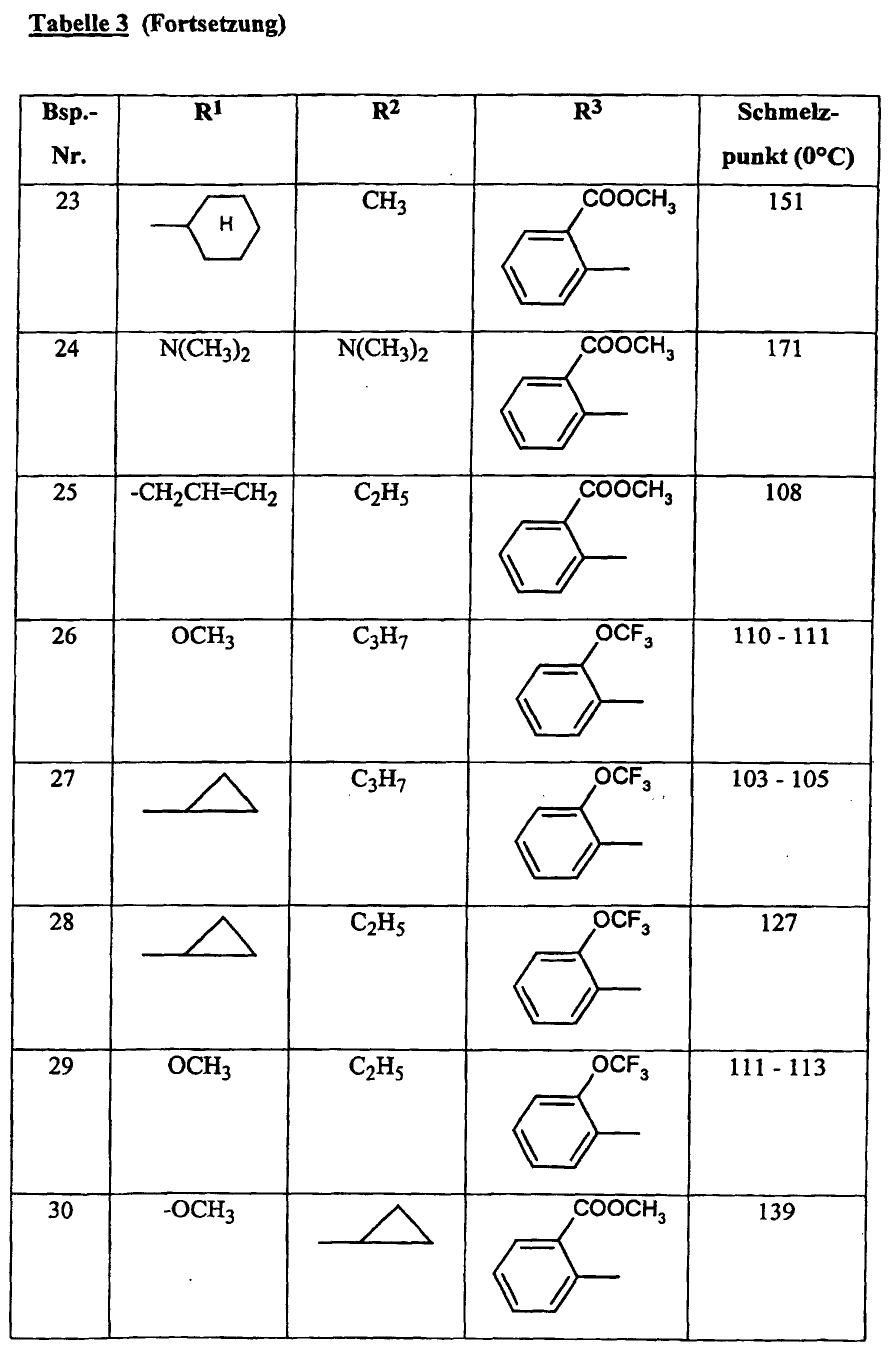

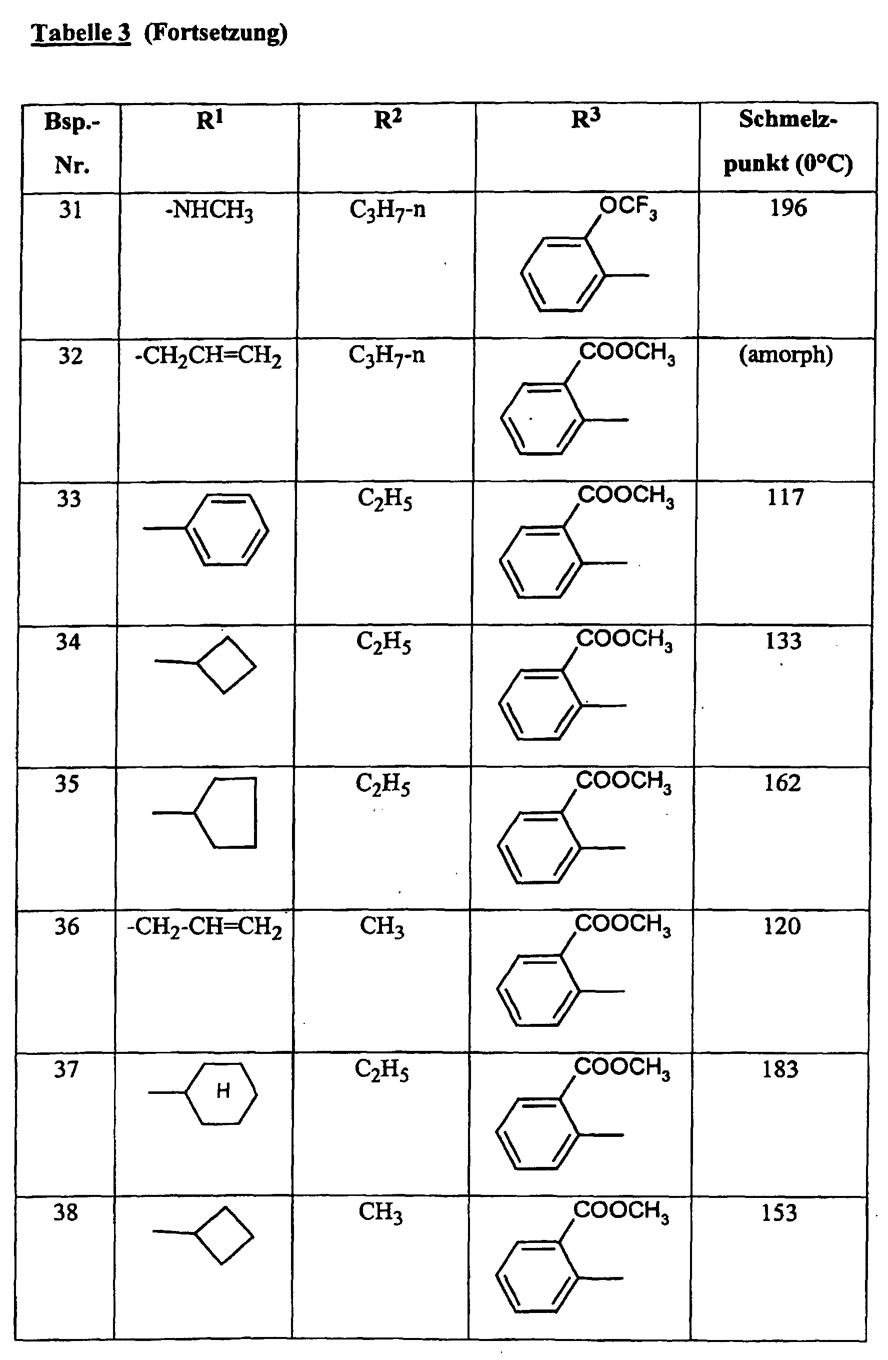

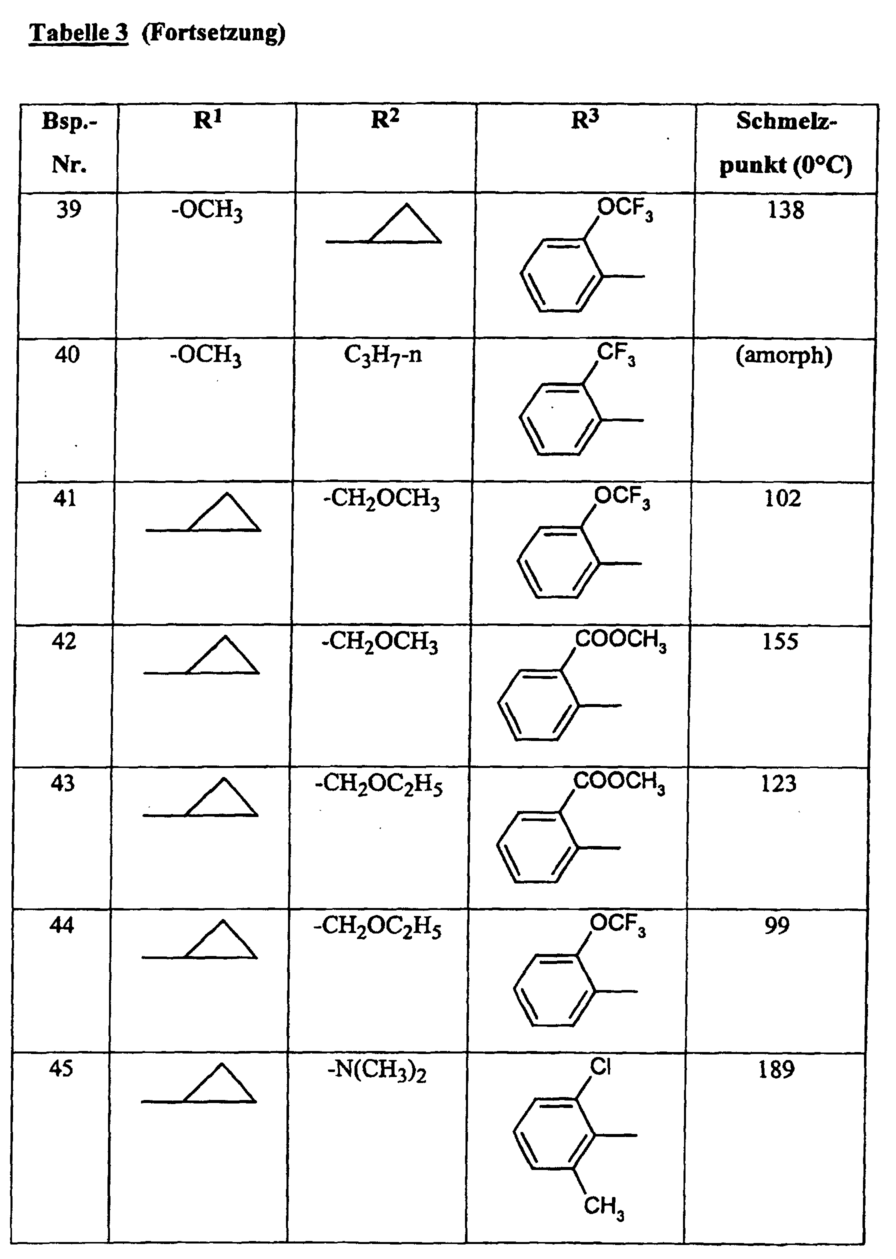

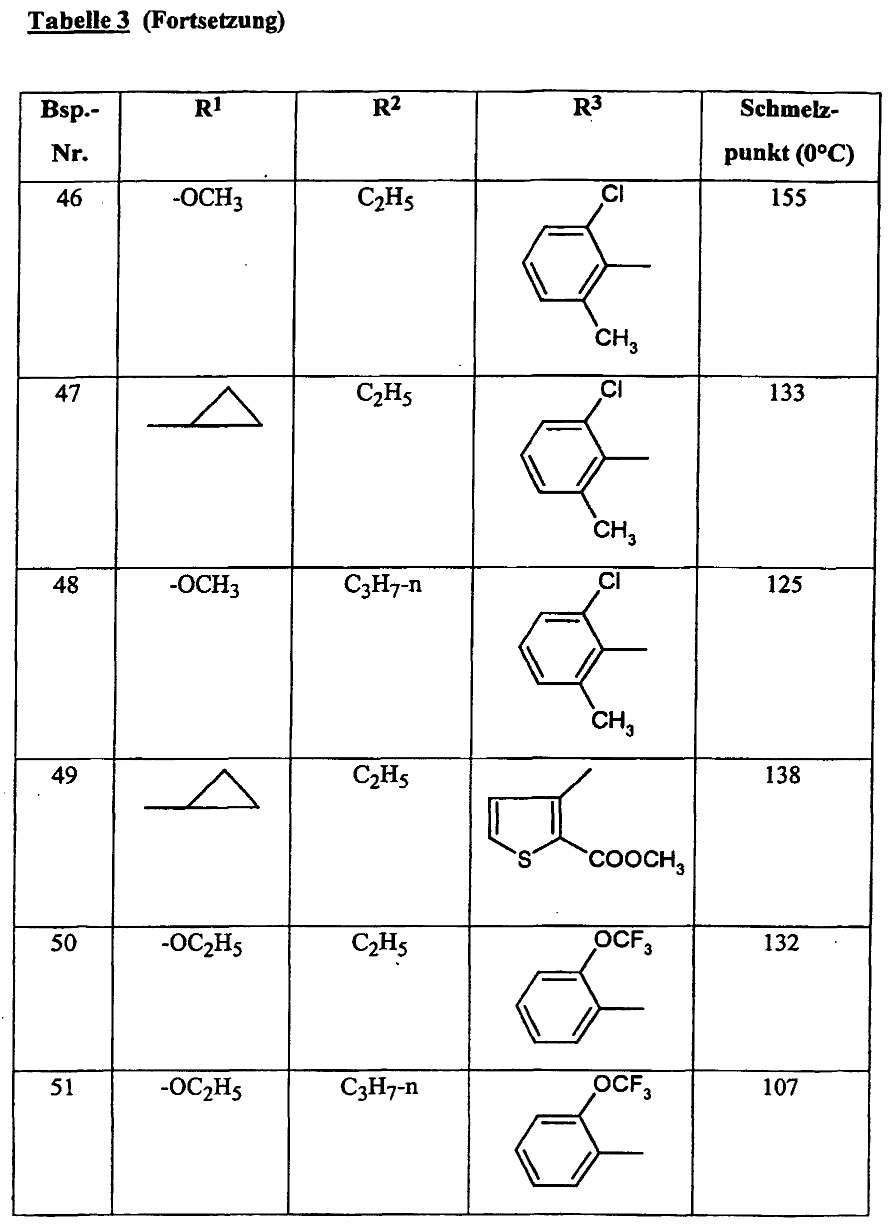

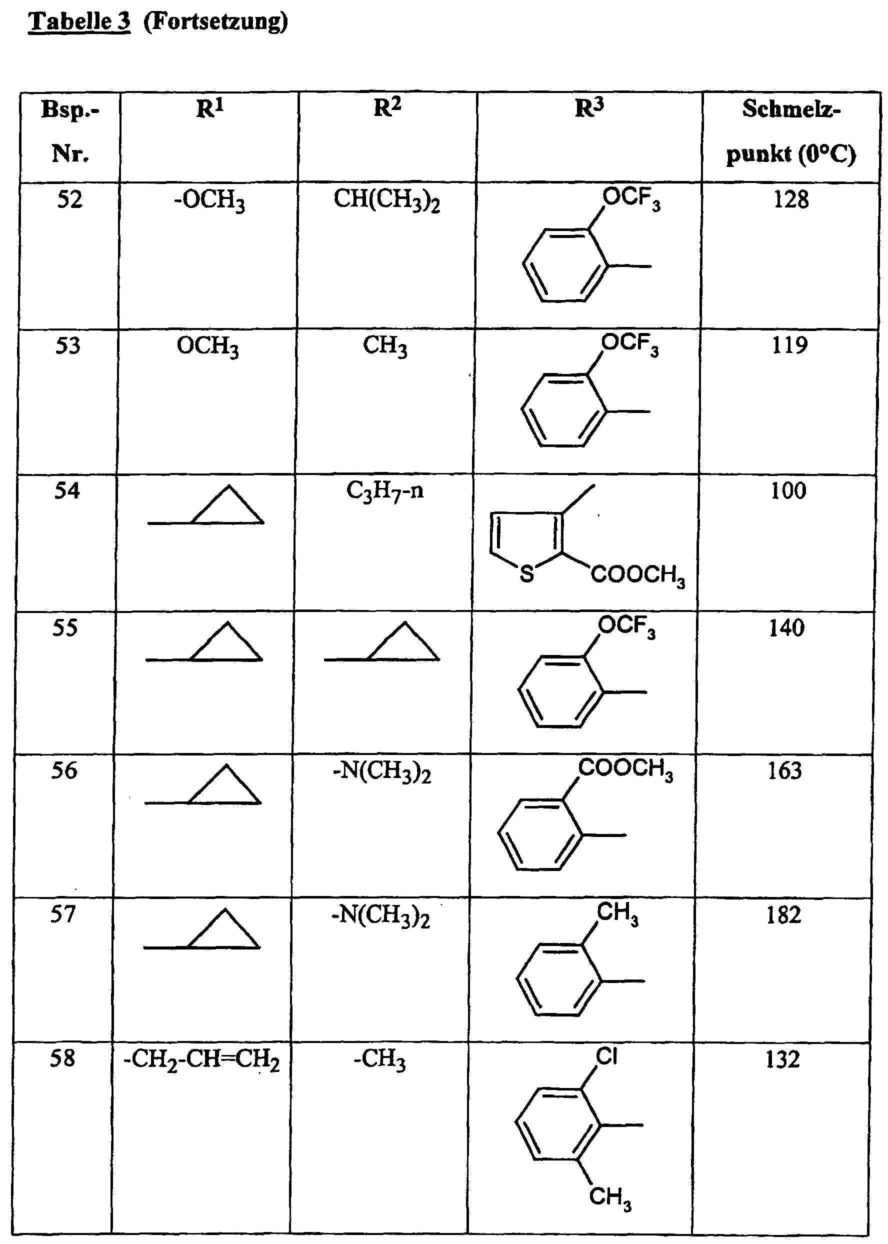

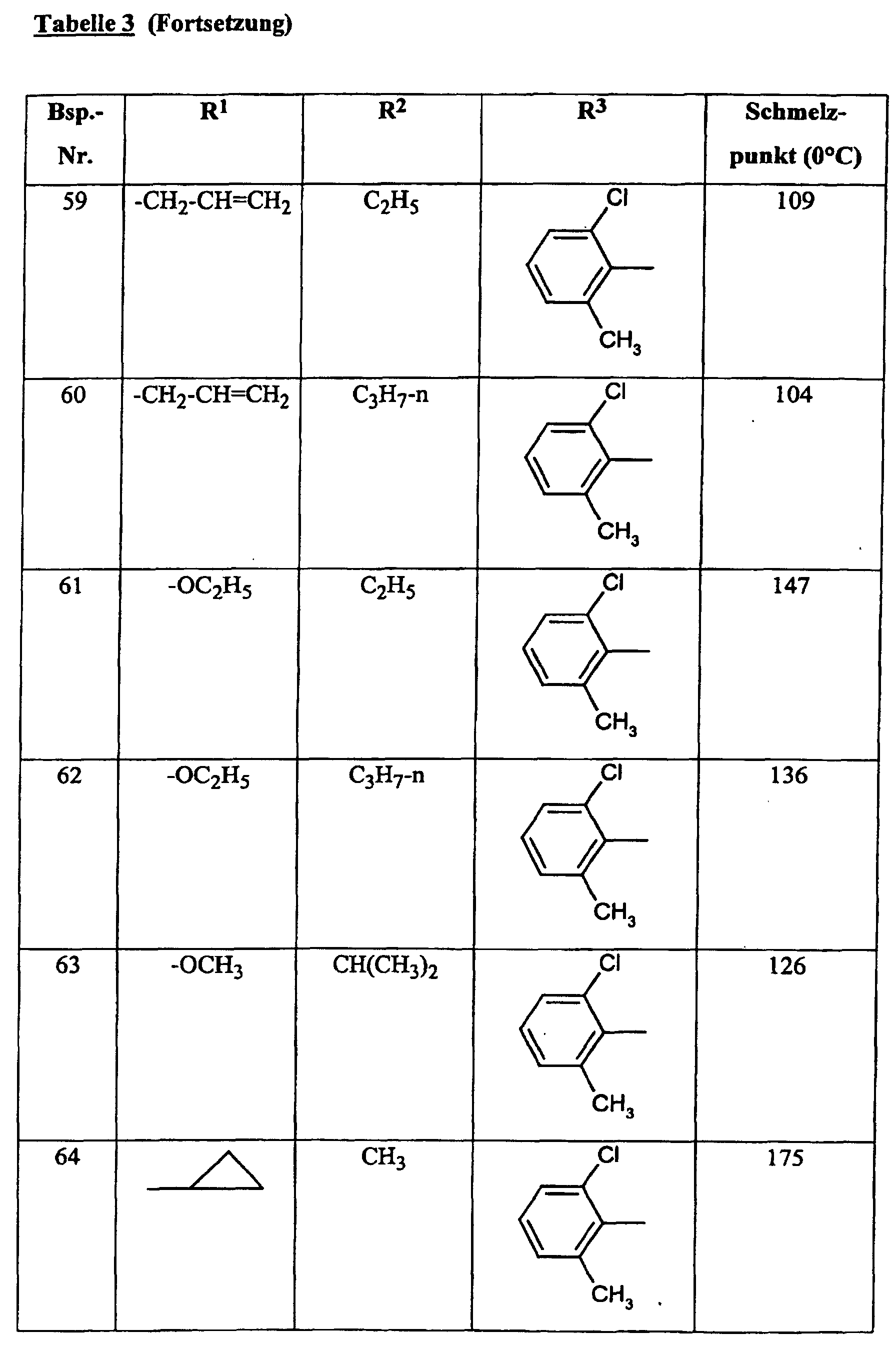

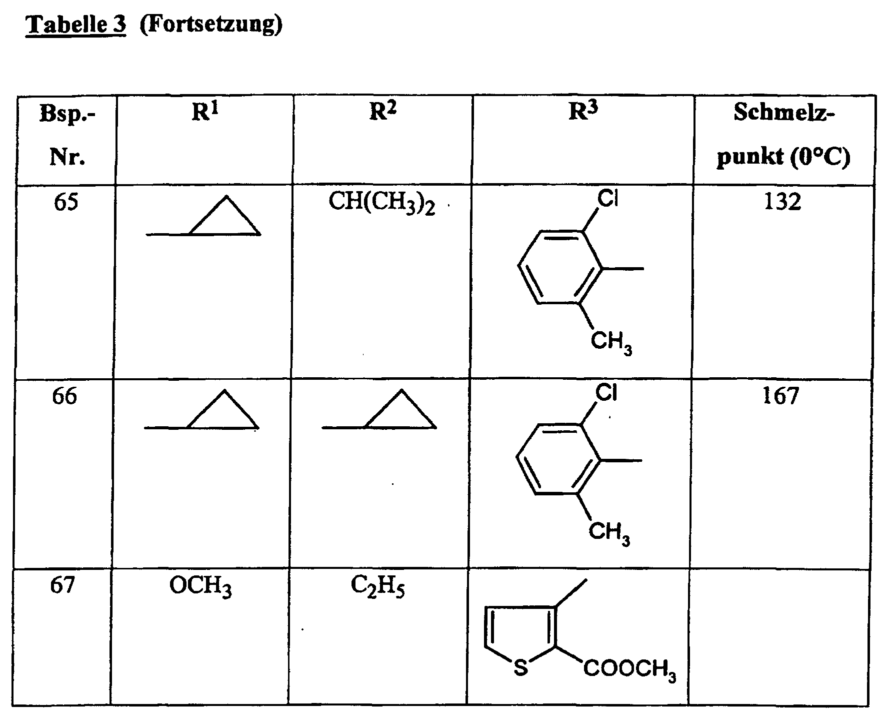

### Ausgangsstoffe der Formel (II):

### Beispiel (II-1)

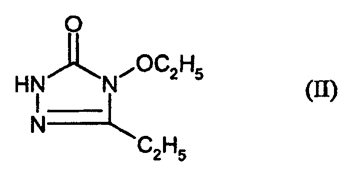

### Stufe 1:

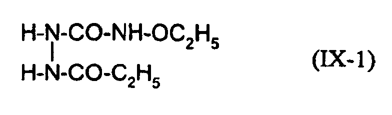

Eine Mischung aus 68,5 g (0,60 mol) 5-Methyl-1,3,4-oxadiazolin-2-on, 45,8 g (0,75 mol) O-Ethyl-hydroxylamin und 400 ml Wasser wird 12 Stunden unter Rückfluss erhitzt und anschließend eingeengt. Der Rückstand wird in Ethanol aufgenommen und erneut eingeengt. Der hierbei erhaltende Rückstand wird mit Diethylether verrührt und das kristallin angefallene Produkt durch Absaugen isoliert.

Man erhält 77,5 g (74 % der Theorie) 1-Ethoxyaminocarbonyl-2-propionyl-hydrazin vom Schmelzpunkt 122°C.

### Stufe 2:

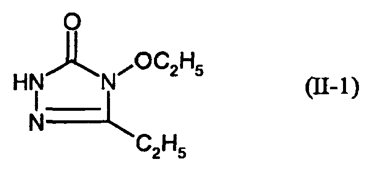

Eine Mischung aus 75,5 g (0,43 mol) 1-Ethoxyaminocarbonyl-2-propionyl-hydrazin, 17,5 g (0,44 mol) Natriumhydroxid und 300 ml Wasser wird 12 Stunden unter Rückfluss erhitzt. Nach dem Erkalten wird durch Zugabe von konzentrierter Salzsäure ein pH-Wert zwischen 3 und 4 eingestellt und eingeengt. Der Rückstand wird mit Essigsäureethylester verrührt und das ungelöst gebliebene Natriumchlorid durch Absaugen abgetrennt. Das Filtrat wird eingeengt, der Rückstand wird mit Diethylether verrührt und das kristallin angefallene Produkt durch Absaugen isoliert.

Man erhält 37 g (55 % der Theorie) 4-Ethoxy-5-ethyl-2,4-dihydro-3H-1,2,4-triazol-3-ön vom Schmelzpunkt 93°C.

Analog zu Beispiel (II-1) können beispielsweise auch die in der nachstehenden Tabelle 4 aufgeführten Verbindungen der Formel (II) hergestellt werden.

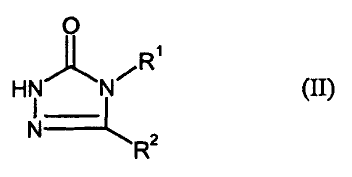

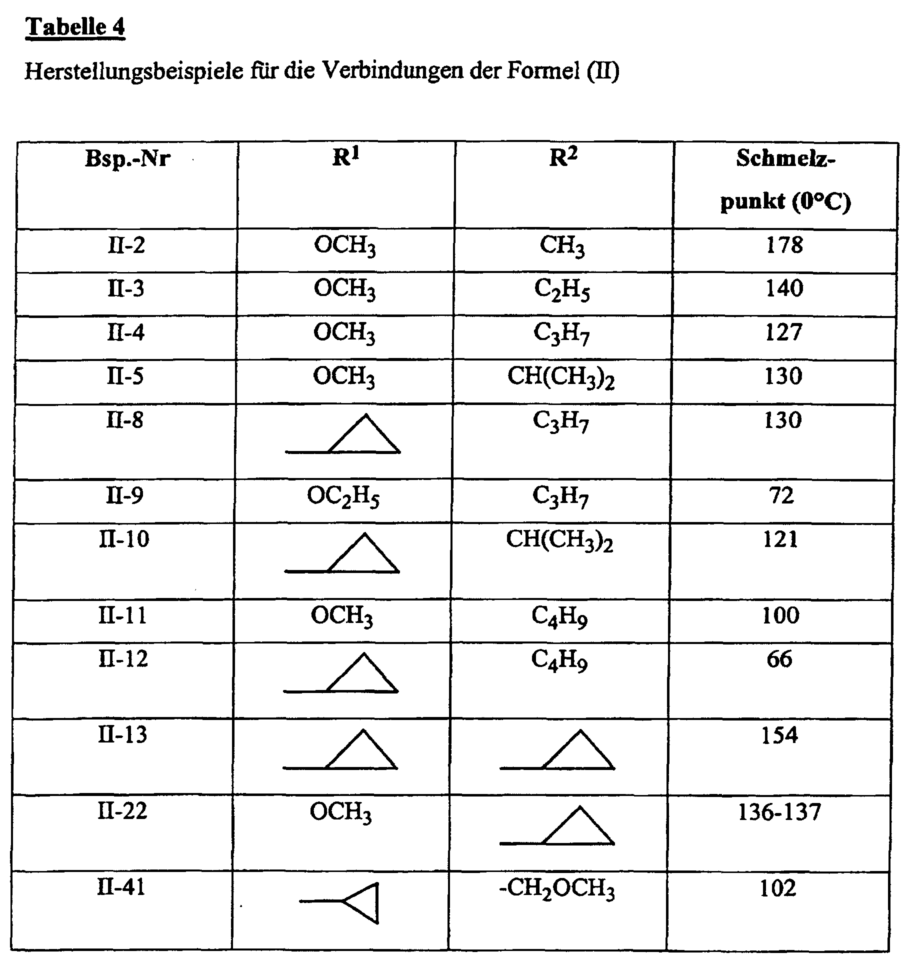

Beispiele für Hydrazinderivate der Formel (IX), welche analog Beispiel (II-1)-Stufe 1 hergestellt werden können, sind in der nachstehenden Tabelle 5 aufgeführt.

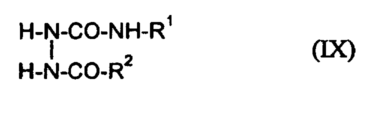

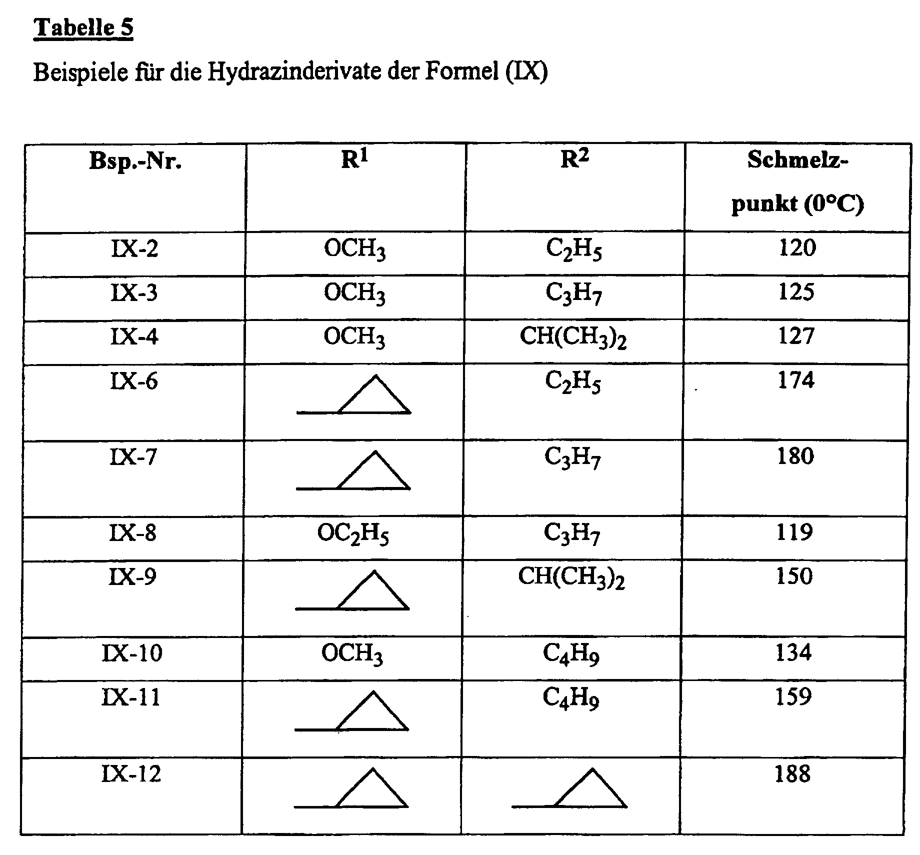

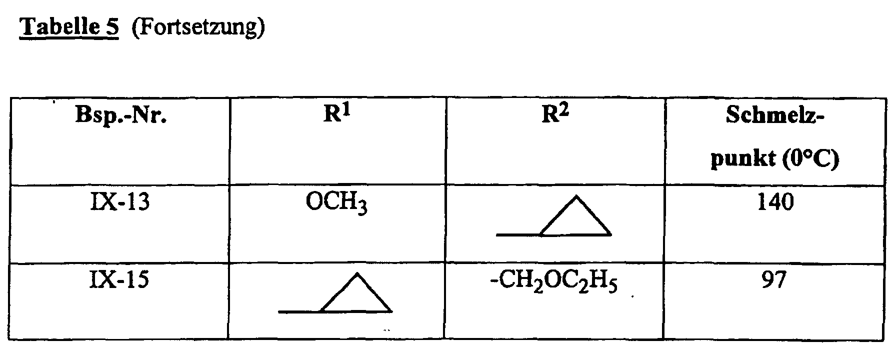

### Anwendungsbeispiele

Bei den folgenden Anwendungsbeispielen wird das bekannte Herbizid Isocarbamid nachstehender Formel (A) als Vergleichssubstanz herangezogen:

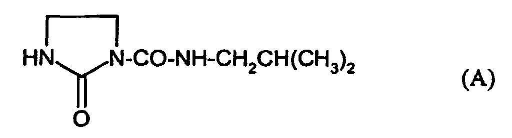

Die Formeln der für die Anwendungsbeispiele herangezogener Verbindungen sind - mit der Nummerierung der Herstellungsbeispiele - nachstehend einzeln aufgeführt:

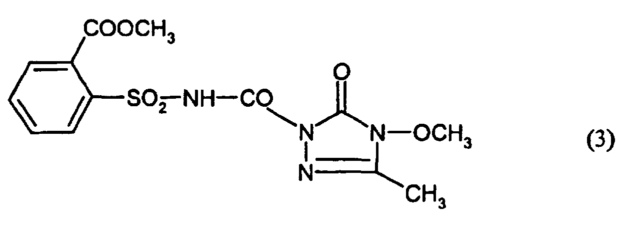

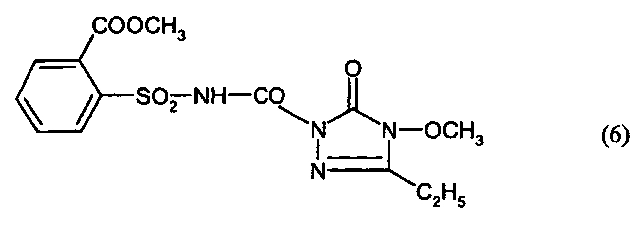

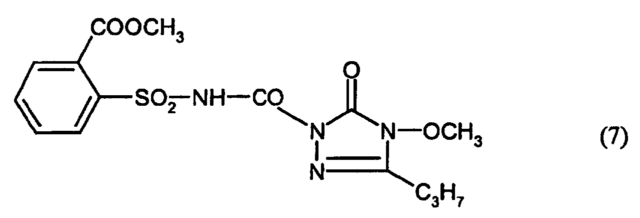

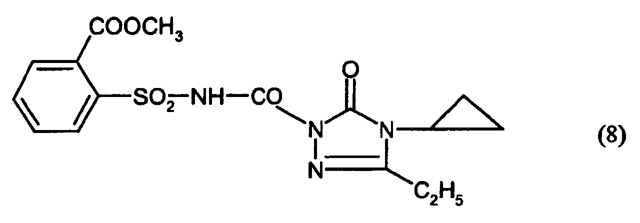

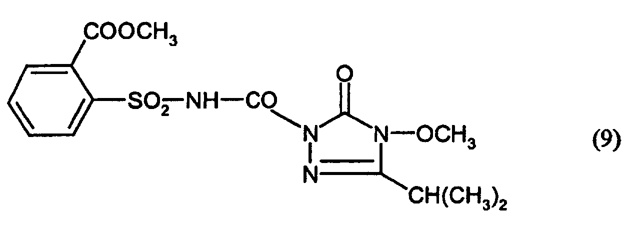

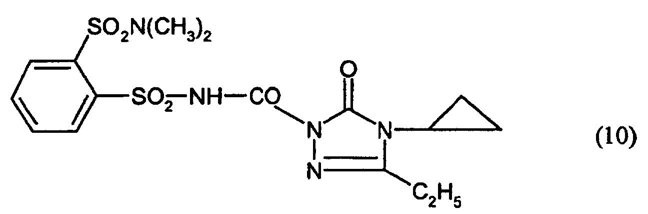

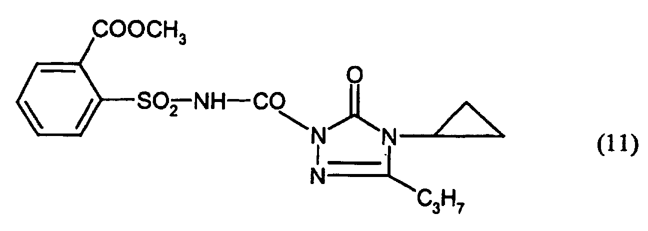

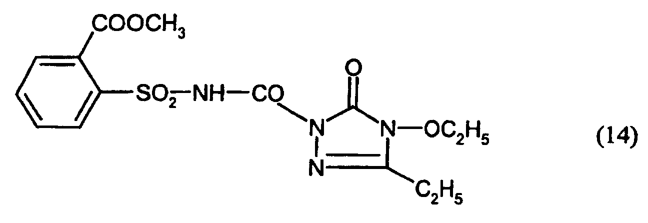

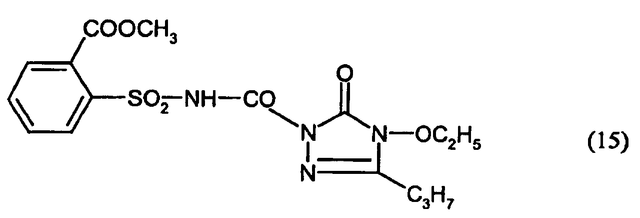

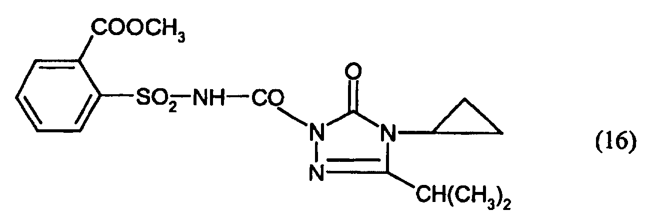

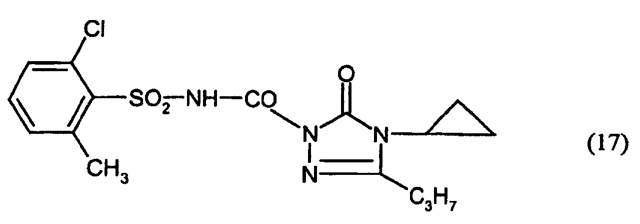

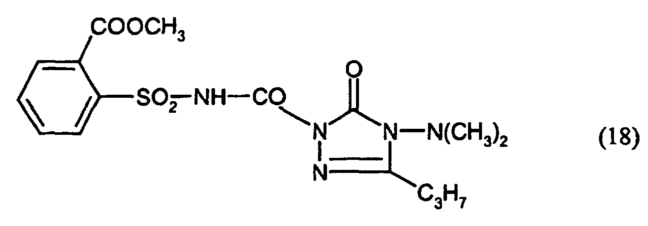

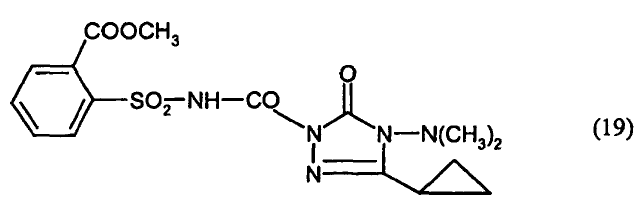

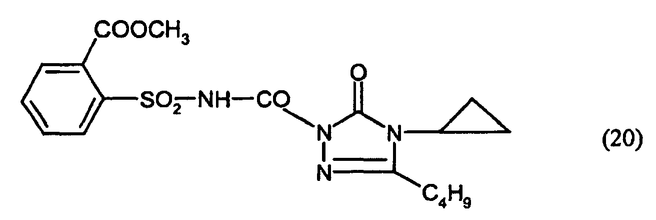

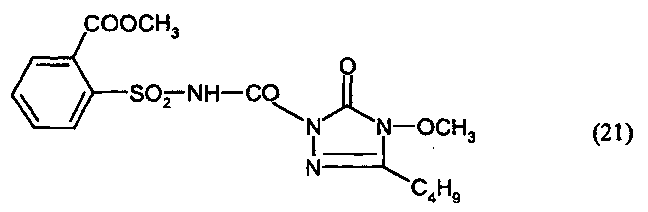

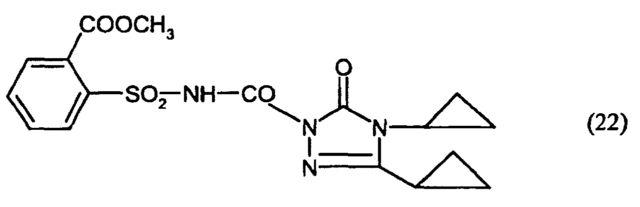

### Beispiel A

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100%= totale Vernichtung Vernichtung

Eine deutliche Überlegenheit in der Wirksamkeit gegenüber dem Stand der Technik zeigen in diesem Test z.B. die Verbindungen gemäß den Herstellungsbeispielen 3, 6, 7, 8, 9, 10, 11, 14, 15, 16, 17 und 22.

### Beispiel B

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, dass die jeweils gewünschten Wirkstoffmengen pro Flächeneinheit ausgebracht werden. Die Konzentration der Spritzbrühe wird so gewählt, dass in 1000 1 Wasser/ha die jeweils gewünschten Wirkstoffmengen ausgebracht werden Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß den Herstellungsbeispielen 6, 7, 8, 9, 10, 11, 14,15, 16, 17, 18, 20 und 22.

### Beispiel C

### Pyricularia-Test (Reis) / protektiv

| | |
|---|---|
| Lösungsmittel | 12,5 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wässrigen Sporensuspension von Pyricularia oryyzae inokuliert. Anschließend werden die Pflanzen in einem Gewächshaus bei 100 % rel. Luftfeuchtigkeit und 25°C ausgestellt.

4 Tage nach der Inokulation erfolgt die Auswertung des Krankheitsbefalls.

Eine gute Wirkung zeigen in diesem Test z.B. die Verbindungen gemäß folgender Herstellungsbeispiele:
3, 6, 7, 18, 19, 20 und 21.

### Beispiel D

### Pyricularia-Test (Reis) / systemisch

| | |
|---|---|
| Lösungsmittel | 12,5 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung aus systemischen Eigenschaften werden 40 ml der Wirkstoffzubereitung auf Einheitserde gegossen, in der junge Reispflanzen angezogen wurden. 7 Tage nach der Behandlung werden die Pflanzen mit einer wässrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine ausgezeichnete Wirksamkeit zeigen in diesem Test z.B. die Verbindungen gemäß folgender Herstellungsbeispiele:
3, 6, 7, 18, 19, 20 und 21.

## Patentansprüche

1. Triazolinone der allgemeinen Formel (II), 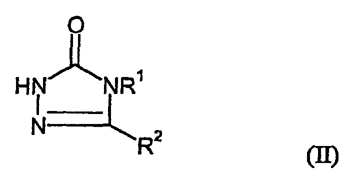 in welcher
R¹ für C₁-C₃-Alkoxy oder Cyclopropyl steht und
(i) wenn R¹ C₁-C₃-Alkoxy bedeutet,
R² für gegebenenfalls durch Fluor und/oder Chlor, Methoxy oder Ethoxy substituiertes C₁-C₄-Alkyl oder für Cyclopropyl steht,
oder
(ii) wenn R¹ Cyclopropyl bedeutet,
R² für Propyl, Isopropyl, Butyl, Cyclopropyl oder Methoxymethyl steht.

2. Triazolinone nach Anspruch 1, in welcher R¹ und R² die in der folgenden Tabelle angegebene Bedeutung besitzen:
| R¹ | R² |
|---|---|
| Methoxy | Methyl |
| Methoxy | Ethyl |
| Methoxy | Propyl |
| Methoxy | Isopropyl |
| Methoxy | Cyclopropyl |
| Ethoxy | Methyl |
| Ethoxy | Ethyl |
| Ethoxy | Propyl |
| Ethoxy | Cyclopropyl |

## Claims

1. Triazolinones of the general formula (II) 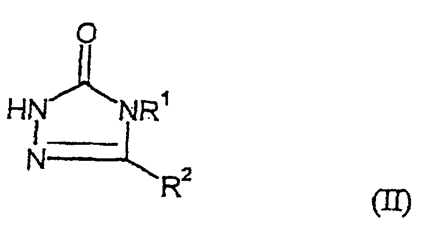 in which
R¹ represents C₁-C₃-alkoxy or cyclopropyl, and
(i) when R₁ is C₁-C₃-alkoxy,
R² represents C₁-C₄-alkyl which is optionally substituted by fluorine and/or chlorine, methoxy or ethoxy, or represents cyclopropyl,
or
(ii) when R₁ is cyclopropyl,
R₂ represents propyl, isopropyl, butyl, cyclopropyl or methoxymethyl.

2. Triazolinones according to Claim 1, in which R¹ and R² have the meanings given in the following table:
| R¹ | R² |
|---|---|
| Methoxy | Methyl |
| Methoxy | Ethyl |
| Methoxy | Propyl |
| Methoxy | Isopropyl |
| Methoxy | cyclopropyl |
| Ethoxy | Methyl |
| Ethoxy | Ethyl |
| Ethoxy | Propyl |
| Ethoxy | Cyclopropyl |

## Revendications

1. Triazolinones de formule générale (II) 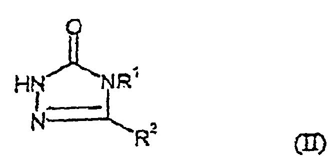 dans laquelle
R¹ est un reste alkoxy en C₁ à C₃ ou un reste cyclopropyle, et
(i) lorsque R¹ est un reste alkoxy en C₁ à C₃,
R² est un reste alkyle en C₁ à C₄ éventuellement substitué par du fluor et/ou du chlore, un radical méthoxy ou éthoxy, ou un reste cyclopropyle, ou bien
(ii) lorsque R¹ est un reste cyclopropyle,
R² désigne un reste propyle, isopropyle, butyle, cyclopropyle ou méthoxyméthyle.

2. Triazolinones suivant la revendication 1, dans lesquelles R¹ et R² ont la définition indiquée dans le tableau suivant :
| R¹ | R² |
|---|---|
| méthoxy | méthyle |
| méthoxy | éthyle |
| méthoxy | propyle |
| méthoxy | isopropyle |
| méthoxy | cyclopropyle |
| éthoxy | méthyle |
| éthoxy | éthyle |
| éthoxy | propyle |
| éthoxy | cyclopropyle |
